(19) **Europäisches Patentamt / European Patent Office / Office européen des brevets**

(11) **EP 1 813 288 B1**

(12) **EUROPEAN PATENT SPECIFICATION**

(45) Date of publication and mention
of the grant of the patent:
**18.07.2018 Bulletin 2018/29**

(51) Int Cl.:
**A61K 9/10** *(2006.01)* **A61K 9/127** *(2006.01)*
**A61K 47/34** *(2017.01)*

(21) Application number: **05807011.1**

(86) International application number:
**PCT/JP2005/021024**

(22) Date of filing: **16.11.2005**

(87) International publication number:
**WO 2006/054589 (26.05.2006 Gazette 2006/21)**

(54) **MEDICINAL COMPOSITION, MEDICINAL PREPARATION, AND COMBINATION PREPARATION**

MEDIZINISCHE ZUSAMMENSETZUNG, MEDIZINISCHE ZUBEREITUNG UND
KOMBINATIONSPRÄPARAT

PRÉPARATION THÉRAPEUTIQUE, FORMULE THÉRAPEUTIQUE, ET FORMULE COMBINÉE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **18.11.2004 JP 2004335040**

(43) Date of publication of application:
**01.08.2007 Bulletin 2007/31**

(73) Proprietor: **Terumo Kabushiki Kaisha
Tokyo 151-0072 (JP)**

(72) Inventors:
- **YOSHINO, Keisuke,**
  **Terumo kabushiki kaisha**
  **Ashigarakami-gun, Kanagawa 259-0151 (JP)**
- **ISOZAKI, Masashi,**
  **Terumo kabushiki kaisha,**
  **Ashigarakami-gun, Kanagawa 259-0151 (JP)**
- **MORIMOTO, Katsumi,**
  **Terumo kabushiki kaisha**
  **Ashigarakami-gun, Kanagawa 259-0151 (JP)**
- **SHIMIZU, Miyuki,**
  **Terumo kabushiki kaisha**
  **Ashigarakami-gun, Kanagawa 259-0151 (JP)**

(74) Representative: **TBK
Bavariaring 4-6
80336 München (DE)**

(56) References cited:
**EP-A1- 1 174 126 WO-A2-2004/078121
WO-A2-2004/096140 JP-A- 04 244 017
JP-A- 07 309 740 JP-A- 07 324 029
JP-A- 11 508 871 JP-A- 2000 510 836
US-A- 5 213 804**

- **YUDA T. ET AL: 'Carier no Bunshi Sekkei: Kecchu Tairyukei Liposome' DRUG DELIVERY SYSTEM vol. 9, no. 3, 1994, pages 145 - 160, XP008118591**
- **KASAOKA T. ET AL: 'Stealth Liposome o Mochiita Delivery System' MEMBRANE vol. 28, no. 3, 2003, pages 135 - 144, XP008036877**
- **JOHNSTONE S.A. ET AL: 'Surface-associated serum proteins inhibit the uptake of phosphatidylserine and poly(ethylene glycol) liposomes by mouse macrophages' BIOCHIMICA. ET BIOPHYSICA. ACTA vol. 1513, 2001, pages 25 - 37, XP004248452**

**Description**

Technical Field

This invention relates to a medicinal composition and preparation, which are useful in drug delivery systems.

Background Art

Recently, extensive studies have been made on drug delivery systems (DDS) wherein a drug is safely and efficiently delivered to and distributed at an intended lesion site. For one of such methods, consideration has been given to the use, as a transporter (carrier) of a drug, a closed vesicle such as a liposome, an emulsion, a lipid microsphere, a nanoparticle and the like. For the practical use of DDS using such a closed vesicle, however, there are many problems to be solved, among which avoidance from the foreign body recognition mechanism of a biologic body and control of disposition are important. More particularly, in order to deliver a closed vesicle to a target site at high selectivity, it is necessary to avoid it from being captured at the reticuloendothelial systems (RES) such as the liver, spleen and the like and prevent aggregation through interaction (adsorption) with the opsonin protein, serum protein and the like, thereby enhancing stability in blood.

For a measure of solving the above problem, there is known membrane modification of a closed vesicle with a hydrophilic polymer. The closed vesicle, particularly liposome, modified with a hydrophilic polymer becomes excellent in retention in blood. This enables the liposome to be passively accumulated at tissues with increased vascular permeability such as tumor tissues, inflammation sites and the like, thereby moving toward practical use. (see patent documents 1 to 3 and non-patent documents 1 to 3). Among them, with regard to a liposome of the type wherein polyethylene glycol (which may be sometimes referred to hereinafter as "PEG") is used as a hydrophilic polymer for modification with PEG, preparation has been realized.

PEG is a linear polymer having a repeat structure of $-(CH_2CH_2O)_n-$. PEG is a polymer that has an amphipathic property and is thus soluble in both water and organic solvents, and is low in toxicity, for which it has been widely applied for stabilization of drugs and improvement of disposition.

Where polyethylene glycol having such characteristic properties is utilized as a surface modifier of a liposome, it is usual to conveniently use polyethylene glycol derivatives wherein PEG and a lipid such as phospholipid, cholesterol or the like are bound. Of these polyethylene glycol derivatives, those that are commercially available and are general-purpose include, for example, polyethylene glycol derivatives bound with diacylphophatidylethanolamine (PEG-PE).

It has been accepted that a drug carrier for supporting a drug in a carrier (e.g. a liposome) modified with PEG, which is known to be low in toxicity, is safe and is able to improve retention in blood.

For an instance where PEG achieves stabilization of drugs, mention is made, for example, of protein preparations. In about 1970, attention was drawn to L-asparaginase, isolated from Escherichia coli bacteria, which gave a remarkable efficacy as a therapeutic agent for leukemia. In this connection, however, ordinary protein preparations involve a problem on in vivo stability and antigenicity, and thus, frequent dosing is impossible with L-asparaginase being no exception. To cope with this, studies on modification with PEG have been in progress as a measure for causing the antigenicity of L-asparaginase to disappear. As a result, PEG-modified L-asparaginase succeeded in the disappearance of antigenicity and enabled frequent dosing to patients, and now serves as a therapeutic agent for leukemia in the United States of America.

It is also known that PEG is able to prevent drugs using proteins from being taken into the liver. For instance, attempts have been made to bind PEG molecules to interferon (used for hepatitis treatment), which is one of protein preparations, for the purpose of improving clinical usefulness. Peginterferon-α-2a preparations (Pegasys Subcutaneous Injection 90 μg, Pegasys Subcutaneous Injection 180 μg), which are one of the attempts, are described in Non-patent Literature 4. Eventually, PEG-modified interferon succeeded in remarkably improving a duration of action with respect to the concentration in blood, and a conventional treatment frequency of dosing three times per week could be reduce to a frequency of dosing once per week, thereby enabling a dosing frequency of a drug using proteins to be reduced. In recent years, developments on therapeutic drugs for thrombocytopenia have been made through fusion with a genetic engineering technique to modify active sites with PEG for improving the in vivo activity.

Patent Document 1: Japanese Unexamined Patent Publication No. Hei 5-505173
Patent Document 2: Japanese Patent Publication No. Hei 7-20857
Patent Document 3: Japanese Patent No. 2667051
Non-patent Document 1: written by D.D. Lasic "LIPOSOMES from Physics to Applications", Elsevier, 1993
Non-patent Document 2: edited by Martin C. Woodle and Gerrit Storm "Long Circulating Liposomes: Old Drugs, New Therapeutics", Springer, 1997

Non-patent Document 3: edited by D.D.Lasic and D. Papahadjopoulos "Medical Applications of LIPOSOMES", Elsevier, 1998

Non-patent Document 4: Drug Interview Form, December, 2003 (third edition), published by Chugai Pharmaceutical Co., Ltd., classification No. of standard drug products; 876399 "Peginterferon-$\alpha$-2a Preparations Pegasys Subcutaneous Injection 90 $\mu$g and Pegasys Subcutaneous Injection 180 $\mu$g"

WO2004078121 (A2) discloses a method for reducing complement activation upon in vivo administration of a liposome preparation containing an entrapped therapeutic agent. The method involves providing liposomes having a polyethylene-glycol-derived neutral lipopolymer.

EP1174126 (A1) mentions a liposome wherein a compound containing a polyalkylene glycol moiety is bonded through a thioether group and an antibody is bonded through a thioether group to a liposome comprising lipids whose partial component has maleimidated terminal, wherein an amount of the bonded compound and an amount of bonded antibody is 15 to 50 mole% and 0.1 to 2 mole%, respectively, based on one mole of the maleimidated lipid contained in the liposome. The liposome achieves both excellent blood retention and therapeutic effect.

US5213804 (A) discloses a liposome composition for localizing an antitumor compound to a solid tumor via the bloodstream. The liposomes, which contain the agent in entrapped form, are composed of vesicle-forming lipids and between 1-20 mole percent of a vesicle-forming lipid derivatized with hydrophilic biocompatible polymer, and have sizes in a selected size range between 0.07 and 0.12 microns. After intravenous administration, the liposomes are taken up by the tumor within 24-48 hours, for site-specific release of entrapped compound into the tumor. In one composition for use in treating a solid tumor, the compound is an anthracycline antibiotic drug which is entrapped in the liposomes at a concentration of greater than about 50 $\mu$g agent/ $\mu$mole liposome lipid. The method results in regression of solid colon and breast carcinomas which are refractory to anthracycline antibiotic drugs administered in free form or entrapped in conventional liposomes.

Disclosure of Invention

Problems to be Solved by the Invention

Such PEG-modified preparations cannot always fully avoid the foreign body recognition mechanism of a biologic body. For instance, it is known that when the blood is sampled, after administration of a PEG-modified drug carrier, so as to check the carrier surface, many proteins are bound to the carrier surface. It has been reported that if rats are readministered after administration of a PEG-modified preparation after a given period of time, there occurs activation of a complement system taking part in an antibody-antigen reaction. Simultaneously, the PEG-modified preparation is rapidly cleared from the blood (ABC phenomenon: Accelerated Blood Clearance). Moreover, it has been confirmed that when readministered with PEG-modified preparations, dogs undergo anaphylactoid/anaphylactic reactions. With pigs, such a reaction as with dogs has been confirmed upon initial administration of PEG-modified preparations. It has been reported that when commercially available PEG-modified liposome preparations are administered to human beings, anaphylactoid/anaphylactic reactions occur at a relatively high frequency, along with the possibility that complement activity takes part in the reactions.

In this way, it has been made clear that various problems associated with such activation of a complement system as set out above are involved in the use of the PEG modified preparations and thus, there is a quick demand for solving these problems. For instance, although an attempt has been made to inhibit the complement activity, for example, by alteration of the structure of the PEG itself, retention in blood is simultaneously impeded, thus not arriving at the resolution of the problems due to this approach.

Currently, a method of PEG binding of a carrier is of general practice in the development of drugs as a DDS preparation, and it is a fact that retention in blood of a DDS preparation is drastically improved by PEG binding to a carrier. Desired retention in blood cannot be attained unless PEG preparations are used. For now, a difficulty is involved in obtaining a satisfactory treating effect without use of PEG-modified preparations.

Fundamentally, a complement is a serum protein that plays an important phylactic role of vertebrates. The complement system is activated through a classical pathway or alternative pathway. In the classical path, an antigen-antibody complex is initially formed, and a complement first component (Clq) is bound to and activated by an Fc portion of the antibody, under which beginning at the activation, the activation reaction of the complement system proceeds. In the alternative pathway, C3 molecules undergoing hydrolysis with polysaccharides such as bacteria react with B and D factors and are activated, so that the activation reaction of the complement system proceeds.

In either of the above paths, C3a/C5a that are complement fragments are formed through the activation of C3/C5 convertases. These are each called anaphylatoxin and act on mast cells and neutrophil to permit inflammatory mediators such as histamine to be freed, thereby causing an increase in vascular permeability and smooth muscle contraction. C5a also acts as a chemotactic factor of neutrophil. Moreover, when C3b, which is a counterpart of C3a, is bound to bacteria or viruses, the opsonina activity is induced wherein the phagocytic capacities of the neutrophil and macropharge are promoted. Accordingly, the biologic activities of the complement system serve mainly to (1) induce bacteriolysis, hemolytic reaction and cytopathic reaction of a membrane invasion complex, (2) induce an inflammatory mediator owing to a complement fragment, and (3) induce the opsonin effect through a complement receptor.

Taking the above into account, it is assumed that for the activation of the complement system with PEG-modified preparations, the decomposition reaction of a complement proceeds by a trigger of binding some complement-activating substance (an antibody, lectin or the like) to a PEG modified preparation, during which anaphylatoxins (C3a and C5a) are formed and cause a series of anaphylactoid/anaphylactic reactions.

Accordingly, an object of the invention is to provide a medicinal composition capable of inhibiting activation of a complement system.

For hitherto known preparations capable of undergoing anaphylactoid/anaphylactic reactions, mention is made, for example, of dextran preparations. The dextran preparation is administered to human beings as a plasma extender, a bloodstream promoter or an antithrombotic agent. It is widely known that dextran-induced anaphylactoid/anaphylactic reactions (DIAR) are a kind of antigen antibody reaction showing from a relatively mild symptom to a lethal symptom, and the occurrence probability of this reaction including all the symptoms is at 0.03 to 4.7%.

For a method of inhibiting this DIAR, mention is made, for example, of pre-administration of a low molecular weight dextran preparation. More particularly, there is, for example, a method wherein immediately before administration of a dextran preparation (with a molecular weight of 40000 (Rheomacrodex) or a molecular weight of 70000 (Macrodex)), 20 ml of dextran having a molecular weight of 1000 (Promit) is administered.

It is known that DIAR is a reaction taking part in the formation of an immunocomplex by binding of IgG to high molecular weight dextran. To cope with the DIAR, low molecular weight dextran is pre-administered to block, with the low molecular weight dextran, a recognition site of IgG relative to a dextran preparation beforehand. It has been elucidated that the administration of the low molecular weight dextran competitively impedes the binding of IgG to a dextran preparation and DIAR can be eventually inhibited (see Joint WHO/IABS Symposium on the Standardization of Albumin, Plasma Substitutes and Plasmapheresis, Geneva 1980, Develop. boil. Standard. 48, pp. 179-189 (S. Karger, Basel 1981)).

Means for Solving the Problems

In order to achieve the above object, we made studies based on the above theory and, as a result, found that when a medicinal composition including a preparation modified with a specific type of hydrophilic polymer, and a specific type of hydrophilic polymer is administered, activation of a complement system can be inhibited, which has been difficult with conventional methods. There has never been reported until now a medicinal composition with which activation of a complement is inhibited by such a method as mentioned above and which has an excellent safety as a drug. Thus, the invention contemplates to provide the medicinal composition defined in claim 1 and the preparation defined in claim 7 so as to solve the above problems. Further beneficial embodiments are disclosed in dependent claims 2 and 8.

Effects of the Invention

The medicinal composition and preparation of the invention are able to inhibit activation of a complement system.

Best Mode for Carrying out the Invention

The invention is described in more detail.

The medicinal composition of the invention is defined in claim 1.

The preparation used in the medicinal composition is illustrated below. The medical composition comprises a liposome made of hydrogenated phosphatidyl choline and cholesterol and whose surface has been modified with a polyethylene glycol derivative, wherein the polyethylene glycol derivative is polyethylene glycol-phosphatidylethanol amine, and has a molecular weight of the polyethylene glycol of the polyethylene glycol derivative of 5000 Dalton, and a polyethylene glycol, wherein the molecular weight of the polyethylene glycol is in the range from 400 to 4000 Dalton. In the following, the first hydrophilic polymer relates to a polyethylene glycol derivative, wherein the polyethylene glycol derivative is polyethylene glycol-phosphatidylethanol amine, and has a molecular weight of the polyethylene glycol of the polyethylene glycol derivative of 5000 Dalton, and the second hydrophilic polymer relates to a polyethylene glycol, wherein the molecular weight of the polyethylene glycol is in the range from 400 to 4000 Dalton. The particle size of the liposome is generally at 0.02 to 1 $\mu$m, preferably at 0.05 to 0.2 $\mu$m.

It will be noted that the outer diameter of the particle is expressed as an average value of diameter of all particles of a liposome preparation measured according to a dynamic light scattering method. In the practice of the invention, the outer diameter of the particle was measured by use of Zetasizer (Malvern Instruments. 3000 HS).

For a liposome modified with a first hydrophilic polymer, mention is made, for example, of one wherein the first hydrophilic polymer is chemically or physically bound to an outside surface of the liposome. As a matter of course, the liposome is able to bind the first hydrophilic polymer to the inside of the liposome. Specific types of liposome include, for example, a liposome of a type wherein the first hydrophilic polymer is bound to both inner and outer sides of the membrane thereof and a liposome of a type wherein the first hydrophilic polymer is bound to only an outer side of the membrane. Of these, a liposome of the type which is selectively surface modified with the first hydrophilic polymer only on the outer side of a lipid bilayer is preferred. This is because with such a liposome, the first hydrophilic polymer ensures the stability of the membrane if the inner water phase is low in pH, thus leading to excellent retention in blood of the liposome.

A liposome is a closed endoplasmic reticulum. More specifically, the liposome has such a structure that a phospholipid containing a hydrophobic group and a hydrophilic group forms a membrane based on both polarities thereof, and the membrane forms a space kept away from outside in the inside thereof. The space has a water phase (inner water phase) therein. The liposome may contain lipids other than a phospholipid as a membrane-constituting ingredient.

The first hydrophilic polymer can be bound to any membrane-constituting ingredients of the liposome.

The liposome should preferably be formed of a lipid bilayer containing a phospholipid as a main membrane material.

The phospholipid is generally an amphipathic substance having a hydrophobic group constituted of a long-chain alkyl group and a hydrophilic group constituted of a phosphate group. The phospholipids include, for example, glycerophospholipids such as phosphatidylcholine (=lecithin), phosphatidylglycerol, phosphatidic acid, phosphatidylethanolamine, phosphatidylserine, and phosphatidylinositol, sphingophospholipids such as sphingomyelin (SM), natural or synthesized diphosphatidyl-based phospholipids and derivatives thereof such as cardiolipin, and those indicated above and hydrogenated by an ordinary method (e.g. hydrogenated soybean phosphatidyl choline (HSPC). These phospholipids may be sometimes referred to hereinafter as "phospholipids".

Of these, hydrogenated phospholipids such as hydrogenated soybean phosphatidyl choline, sphingomyelin and the like are preferred.

For a liposome, it is preferred to use, as a main membrane material, a phospholipid whose phase transition point is higher than an in vivo temperature (35 to 37°C). This is because the use of such a phospholipid enables a drug entrapped in the liposome not to be readily leaked from the liposome to outside during storage or in a living subject such as a blood. The liposome may be, in some case, exposed to a temperature higher than a living body temperature during the course of preparation. More particularly, liposomes may be prepared under temperature conditions, for example, of about 50 to 70°C, more particularly, about 60°C. The temperature higher than the living body temperature greatly influences the formation of a liposome. The phase transition point of the main membrane material of the liposome should preferably be at a level higher than a preparation temperature thereof or at a temperature exceeding the preparation temperature. More particularly, the phase transition point of the main membrane material is at 50°C or over as a preferred embodiment.

The liposome may contain a single type of phospholipid or plural types of phospholipids.

The method of modifying a liposome with a first hydrophilic polymer is accorded to a hitherto known procedure. For instance, mention is made of a method wherein a phospholipid and a lipid derivative serving as a first hydrophilic polymer are preliminarily, uniformly mixed to form a liposome (pre-introduction method), a method wherein after formation of a liposome of a lipid bilayer, a lipid derivative of a first hydrophilic polymer is added thereby modifying the membrane surface of the liposome with the lipid derivative of the first hydrophilic polymer from outside (post-introduction method), and a method of preparing a liposome modified with a first hydrophilic polymer where after the preparation of a liposome containing a membrane constituting lipid such as a phospholipid having a reactive functional group in a usual manner, a first hydrophilic polymer activated at one end thereof is added to a liposome dispersion for binding with a membrane constituting lipid having a functional group such as a phospholipid.

Of these, the post-introduction method is a preferred one. This is because according to this method, there can be obtained a liposome that is selectively surface modified with the first hydrophilic polymer only at the outer side of the lipid bilayer. The resulting liposome is in such a state that the first hydrophilic polymer moiety projects outwardly of the lipid bilayer and the lipid moiety serving as a hydrophobic portion is stably held as entering into the lipid bilayer of the liposome.

The lipid derivative of the first hydrophilic polymer is a derivative made of the first hydrophilic polymer and a lipid.

The first hydrophilic polymer is described below.

The first hydrophilic polymer is not critical in type. Hitherto known hydrophilic polymers can be used.

Of there, preferred first hydrophilic polymers are ones having, as a unit structure, at least one unit selected from the group consisting of $-CH_2CH_2O-$, $-CH_2CH_2CH_2O-$, $-[CH_2CH(OH)CH_2O]_n-$ and $-CH_2CH(CH_2OH)O-$.

It will be noted the unit structure used herein means a minimum unit constituting the main chain of a first hydrophilic polymer or second hydrophilic monomer. Where two or more of the same units are combined to form the main chain of the first hydrophilic polymer or second hydrophilic polymer, the resulting unit structure corresponds to a generally called recurring units.

The unit structure should preferably have at least one member selected from the group consisting of-$(CH_2CH_2O)_n$-, -$(CH_2CH_2CH_2O)_n$-, - $[CH_2CH(OH)CH_2O]_n$- and-$[CH_2CH(CH_2OH)O]_n$-. In the formula, n is an integer of not smaller than 1, preferably 1 to 1,000.

For the first hydrophilic polymer, mention is made, for example, of a homopolymer made of one type of unit structure, and copolymers, terpolymers, and block copolymers, respectively, formed of two or more unit structures. Of these, homopolymers are preferred. The reason for this is that because the invention is characterized in that the recognition of the first hydrophilic polymer with a complement activating substance is competitively inhibited with the second hydrophilic polymer, the structure of the first hydrophilic polymer should favorably be one, with which the complement activating substance can be simply appreciated.

Specific examples of the first hydrophilic polymer include polyethylene glycols, polyglycerines, polypropylene glycol, ficoll, polyvinyl alcohol, a styrene-maleic anhydride alternate copolymer, a divinyl ether-maleic anhydride alternate copolymer, polyvinylpyrrolidone, polyvinyl methyl ether, polyvinylmethyloxazoline, polyethyloxazoline, polyhydroxypropyloxazoline, polyhydroxypropylmethacrylamide, polymethacrylamide, polydimethylacrylamide, polyhydroxypropyl methacrylate, polyhydroxyethyl acrylate, hydroxymethyl cellulose, hydroxyethyl cellulose, polyaspartamide, synthetic polyamino acid and the like.

Of these, because of the excellent effect of retention in blood of preparations, polyethylene glycols, polyglycerines and polypropylene glycols are preferred, among which polyethylene glycol (PEG), polyglycerine (PG) and polypropylene glycol (PPG) are more preferred. It is to be noted that such a first hydrophilic polymer is preferably one wherein the terminal end at which no lipid is bound is alkoxylated (e.g. methoxylated, ethoxylated or propoxylated). The reason for this resides in the excellence in storage stability.

It will be noted that in the practice of the invention, the "retention in blood" means the property of a drug existing in the blood as included in a drug carrier, for example, in a host administered with the drug carrier. When a drug is released from a drug carrier, it quickly disappears in the blood and is subjected to exposure. Good retention in blood enables a drug to be administered in smaller amounts.

In the practice of the invention, the "exposure" means the action of a drug, released to outside of a drug carrier, on an external environment. More particularly, the released drug comes close to and contacts with a target site to locally act on cells in a cell cycle phase where DNA synthesis of the target site is carried out, thus showing an expected effect (e.g. an antitumor effect). In order to show such an effect, it is necessary to balance a release rate of a drug from a drug carrier and a retention in blood of the drug carrier. The release rate will be described hereinafter.

The molecular weight of PEG in the present invention is defined in claims 1 and 7. The molecular weight of PEG generally ranges 500 to 10,000 daltons, preferably 1,000 to 7,000 daltons and more preferably 2,000 to 5,000 daltons. The molecular weight of PPG is not critical. The molecular weight of PPG generally 100 to 10,000 daltons, preferably 200 to 7,000 daltons and more preferably 1,000 to 5,000 daltons.

The lipid used for binding to the first hydrophilic polymer is now described below.

The lipid is not critical in nature. For instance, mention can be made of compounds (hydrophobic compounds) having a hydrophobic region. For hydrophobic compounds, mention can be made, for example, of phospholipids constituting such mixed lipids, other types of lipids such as sterols, or long-chain aliphatic alcohols, polyoxypropylene alkyl, glycerine fatty acid esters and the like. Of these, phospholipids are preferred ones.

The acyl chain contained in the phospholipids should preferably be a saturated fatty acid. The chain length of the acyl chain is preferably $C_{14}$ to $C_{20}$, more preferably $C_{16}$ to $C_{18}$. Examples of the acyl chain include dipalmitoyl, distearoyl and palmitoylstearoyl.

The phospholipids are not critical in type. For a phospholipid, there can be used, for example, those having a functional group capable of reaction with the first hydrophilic polymer. Specific examples of the phospholipid having a functional group capable of reaction with the first hydrophilic polymer include phosphatidylethanolamine having an amino group, phosphatidylglycerol having a hydroxyl group, and phosphatidylserine having a carboxy group. The use of the phosphatidylethanolamine is realized in the present invention as defined in the claims.

The lipid derivative of the first hydrophilic polymer is made of the first hydrophilic polymer and such a lipid as indicated above. The combination of the first hydrophilic polymer and the lipid is not critical. Depending on the purpose, an appropriate combination can be used. For instance, mentions is made of derivatives of first hydrophilic polymers wherein at least one selected from phospholipids, other types of lipids such as sterols, long-chain fatty acid alcohols, polyoxypropylenealkyl, and glycerine fatty acid esters and at least one selected from PEG, PG and PPG are bound together. Where the first hydrophilic polymer is made of polyethylene glycol (PEG), it is preferred to select a phospholipid or cholesterol as a lipid. For a lipid derivative of PEG made of such a combination as indicated above, mentions is made, for example, a phospholipid derivative of PEG and a cholesterol derivative of PEG.

Of these, the phospholipid derivative of PEG is one of preferred examples. For a phospholipid derivative of PEG, mention is made, for example, polyethylene glycol-distearoyl phosphatidylethanolamine (PEG-DPSE). PEG-DSPE is preferred because it is a general-purpose compound and is readily available.

The first hydrophilic polymers may be used singly or in combination of two or more.

The lipid derivatives of the first hydrophilic polymer can be prepared by hitherto known methods. For a method of synthesizing a phospholipid derivative that is an < instance of a lipid derivative of the first hydrophilic polymer, mention is made, for example, of a method wherein a phospholipid having a functional group capable of reaction with PEG and PEG are reacted by use of a catalyst. The catalysts include, for example, cyanuric chloride, a carbodiimide, an acid anhydride, and a glutaldehyde. According to the reaction, there can be obtained a phospholipid derivative of PEG wherein the function group and PEG are covalently bonded.

The liposome surface modified with the lipid derivative of the first hydrophilic polymer can prevent an opsonin protein and the like in the blood plasma from adsorption on the surface of the liposome to enhance the stability in blood of the liposome, thereby avoiding capture with RES. For this, deliverability of a drug to an intended tissue or cells is enhanced.

The liposome may further contain, aside from the phospholipid derivative as the lipid derivative of the first hydrophilic polymer, other types of membrane ingredients. Other membrane ingredients include, for example, lipids other than phospholipids and derivatives thereof (which may be hereinafter referred to sometimes as "other lipids"). Liposome should preferably be formed of a membrane of a mixed lipid containing, as a main membrane material, other type of lipid along with the phospholipid and a lipid derivative of the first hydrophilic polymer.

Lipids other than the phospholipid have a hydrophobic group constituted of a long chain alkyl group or the like in the molecule and contains no phosphoric acid group in the molecule. The lipids are not critical in type. Mention is made, for example, glyceroglucolipids, sphingolipids, sterols such as cholesterol, and derivatives such as hydrogenated products thereof. Sterols are not critical in type provided that they have a cyclopentahydrophenanthrene ring. For instance, mention is made of cholesterol.

Liposome may contain such other types of membrane ingredients singly or in combination.

Liposome may further contain, aside from such membrane ingredients as set out above, other type of membrane ingredient within a range not impeding the purpose of the invention. Other type of membrane ingredient is not critical provided that it is able to keep a membrane structure and is able to contain a liposome. Such other type of membrane ingredient includes, for example, a surface modifier. The surface modifier is one which changes physical properties of lipids and impart desired characteristic properties to the membrane ingredients of the carrier, and is not critical in type. For instance, mention is made of charge substances, and water-soluble polysaccharides and derivatives thereof.

The charge substances are not critical in type. For instance, mention is made of compounds having a basic functional group such as an amino group, an amidino group or a guadinino group, and compounds having an acidic functional group.

Examples of the basic compounds include, for example, DOTMA set out in Japanese Patent Laid-open No. Sho 61-161246, DOTAP indicated in Japanese Unexamined Patent Publication No. Hei 5-508626, transfectam set out in Japanese Patent Laid-open No. Hei 2-292246, TMAG set out in Japanese Patent Laid-open No. Hei 4-108391, 3,5-dipentadecyloxybenzamidine hydrochloride, DOSPA, DOTAP, TfxTM-50, DDAB, DC-CHOL, DMRIE and the like set out in the pamphlet of WO97/42166.

The lipid derivatives wherein such a compound having a basic functional group as mentioned above and a lipid are bound together is called a cationized lipid. The lipid is not critically limited in type. For instance, mention is made of a compound having a hydrophobic region (a hydrophobic compound). The hydrophobic compound includes, for example, phospholipids constituting such a mixed lipid as mentioned above, other lipids such as sterols, long-chain aliphatic alcohols, polyoxypropylene alkyls, glycerine fatty acid esters and the like. Of these, the phospholipid is one of preferred examples. Where the liposome has a cationized lipid as a surface modifier, the cationized lipid is stabilized in the lipid bilayer of the liposome and permits the basic functional group moiety to exist on the membrane surface (at least on an outer membrane surface or inner membrane surface) of the lipid bilayer. The modification of the membrane with the cationized lipid enables adhesion between the liposome membrane and cells to be increased.

For compounds having an acidic functional group, mention is made, for example, of acidic phospholipids such as phosphatidinic acid, phosphatidylcerine, phosphatidylinocitol, phosphatidylglycerol, and cardiolipin, saturated or unsaturated acids such as oleic acid and stearic acid, sialic acids such as ganglioside GM1 and ganglioside GM3, and acidic amino acid-based surface active agents such as N-acyl-L-glutamine.

Water-soluble polysaccharides are not critically limited. Mention is made, for example, of glucuronic acid, sialic acid, dextran, pullan, amylase, aminopectin, chitosan, mannan, cyclodextrin, pectine, carrageenan and the like. The derivatives of the water-soluble polysaccharide includes, for example, glucolipids.

If the surface modifier contains a water-soluble polysaccharide, the lipid bound to the water-soluble polysaccharide includes, for example a compound (hydrophobic compound) having a hydrophobic region. Examples of the hydrophobic compound include phospholipids constituting such a mixed lipid as mentioned above, other type of lipids such as sterols, long-chain aliphatic alcohols, polyoxypropylene alkyls, glycerine fatty acid esters and the like. Of these, the phospholipid is one of preferred examples.

The surface modifiers may be used singly or in combination of two or more.

In the liposome modified with the first hydrophilic polymer, the rate of modification of the lipid membrane (total lipids) with the first hydrophilic polymer is generally at 0.1 to 20 mol%, preferably 0.1 to 5 mol% and more preferably 0.2 to 10 mol% relative to the liposome membrane lipid.

Where PEG is used as the first hydrophilic polymer, the rate of modification of the lipid membrane (total lipids) with PEG is generally at 0.1 to 20 mol%, preferably 0.1 to 10 mol% and more preferably 0.2 to 10 mol% relative to the liposome membrane lipids.

It will be noted that in the practice of the invention, the "total lipids" means a total amount of all lipids for the liposome membrane containing a lipid derivative with the first hydrophilic polymer. The lipids include, for example, phospholipids, lipids of the lipid derivatives with first hydrophilic polymers, other types of lipids, and lipids contained in surface modifiers.

In the liposome modified with the first hydrophilic polymer, the amount of a phospholipid serving as a main component is generally at 20 to 100 mol%, preferably 40 to 100 mol%, of the total membrane lipids.

In the liposome modified with the first hydrophilic polymer, the amount of lipids other than the phospholipids is generally at 0 to 80 mol%, preferably 0 to 60 mol%, of the total membrane lipids.

The method of preparing a liposome used in the invention is not critically limited. The liposome can be prepared according to a hitherto known method. Using such components as stated hereinbefore, liposomes can be prepared by any known methods including, for example, an ethanol injection method, a thin film method, a reverse phase evaporation method, a freeze-thaw method, an ultrasonic method, a high pressure discharge method, an extrusion method, a supercritical method and the like. According to the high pressure discharge method, a high pressure discharge emulsifying machine is used for high pressure discharge to obtain a liposome. This method is particularly reported in "Liposomes in Life Science" (by Terada, Yoshimura et al, Springer/Fairlark, Tokyo (1992)).

As a method for sizing liposome to a desired size, publicly known conventional technology can be available. For example, "Liposome Technology Liposome Preparation and Related Techniques" 2nd edition, Vol. I-III, G. Gregoriadis, editor. CRC Press.

With respect to the lipid bilayer of a liposome, there are known unilamellar vesicles (a small unilamellar vesicle SUV, a large unilamellar vesicle LUV) and a multilamellar vesicle MLV) made of plural lamellae. In the invention, either of the membrane structures may be used for the liposome. Of these, a liposome of a unilamellar structure is preferred and in view of the stability of a liposome, a LUV liposome is preferred.

The unilamellar vesicle liposome can be prepared according to a hitherto known method. For instance, a liposome is dispersed in a solvent such as water-free methanol to provide a liposome dispersion of a liposome constituted mainly of a multilamellar liposome having a multi-layered structure. Next, this liposome dispersion is forcedly passed through filters plural times using an extruder to obtain a unilamellized liposome. Usually, the filters used include two or more filters whose pore diameters differ from one another and which include a filter whose pore diameter is larger than a desired one and a filter having a finally desired pore diameter. When using an extruder at greater times of passages through filters having different pore diameters, a high rate of unilamellaization is attained, thereby obtaining those regarded as a liposome of a substantially unilamellar vesicle. The "substantially unilamellar vesicle" means that the ratio of the unilamellar vesicle in the total carrier (vesicles) for a liposome preparation is at not smaller than 50% of the total as an abundance ratio, preferably not smaller than 80%.

For a drug supported in the carrier, drugs for treatment and/or diagnostics are included. Specific examples of the drug for treatment supported in the carrier include nucleic acid, polynucleotides, genes and analogs, anticancer drugs, antibiotics, exogenous enzymes, antioxidants, lipid inhibitors, hormone drugs, steroid drugs, antiflammatory drugs, steroid drugs, vasodilators, angiotensin-converting enzyme inhibitors, angiotensin receptor antagonists, proliferation and migration inhibitors for smooth muscle cells, platelet aggregate inhibitors, anticoagulants, migration inhibitors of chemical mediators, growth promoters or inhibitors of vascular endothelial cells, aldose reductase inhibitors, mesangial cell proliferation inhibitors, lipoxygenase inhibitors, immune inhibitors, immune activators, antiviral drugs, Maillard reaction inhibitors, amyloidosis inhibitors, nitrogen monoxide synthesis inhibitors, AGEs (advanced glycation endproducts) inhibitors, drugs used for photochemical therapy, drugs used for neutron capturing therapy, drugs used for acoustic-chemical therapy, drugs used for thermotherapy, radical scavengers, proteins, peptides, glycosaminoglycan and derivatives thereof, oligosaccharides, and polysaccharides and derivatives thereof.

Of these, dopamine hydrochloride, gabexate mesylate, norepinephrine, bromhexine hydrochloride, metoclopramide, epinephrine, vitamin B1, vitamin B6, carboplatin, cisplatin, oxaliplatin, doxorubicin hydrochloride, epirubicin hydrochloride, gemcitabine hydrochloride, irinotecan hydrochloride, topotecan hydrochloride, vinorelbine tartarate, and vincristine sulfate are preferred.

The drugs for diagnostics supported in the carrier are not critical in type unless they impede the formation of a drug carrier. More particularly, mentions is made, for example, of in vivo drugs such as radiographic contrast agents, ultrasound diagnostic agents, radiolabelling nuclear medicine diagnostic agents, diagnostic agents for nuclear magnetic resonance and the like.

Supporting of a drug in the carrier is feasible by any hitherto known methods. After-treatment for preparation of a drug carrier is not critically limited. For instance, one of preferred embodiments should be through a stage where a drug not included is removed from the resulting processed liquid. In this condition, a concentration gradient of a drug occurs between the inside and outside of the drug carrier through the lipid bilayer. It is preferred that the drug carrier is not included inside the lipid double layer and thus, a drug is not left outside the lipid bilayer after preparation.

The thus obtained drug carrier is administered to a living body, whereupon it arrives at a target site in a condition where the drug is included, resulting in the delivery of the drug to the target site. The drug carrier permits the included drug to be released to an outside environment. The deliver of the drug to the target site may be such that the drug supported in the carrier is taken in the target site, or an influence of the drug is given to the target site or its vicinity without being taken in the target site.

In the practice of the invention, the "release" means that the drug included in a drug carrier is exposed to outside of the carrier by passage through a lipid membrane for the carrier or by change of the structure of part of the lipid membrane.

The "release rate" means a ratio (%) of an amount of a drug exposed to outside of the carrier from the drug carrier within a given period after administration relative to a total amount of the drug supported in the administered drug carrier. The "release rate is low" means that an amount of a drug exposed to outside of the carrier per unit time is small.

Next, the physiologically active substance modified with the first hydrophilic polymer is now illustrated.

The first hydrophilic polymer used for modifying a physiologically active substance is similar to the first hydrophilic polymer set forth hereinbefore.

The physiologically active substance is not critical in type. Mention is made of proteins, porphyrin complexes such as chlorophyll, chlorophid and the like, porphyrin halides such as hemin, vitamins, antibiotic substances, neutrotransmitters, anticancer agents, nucleic acids, polynucletides, genes and analogs thereof, exogenous enzymes, coenzymes, antioxidants, lipid inhibitors, hormone drugs, antiflammatory drugs, steroid drugs, vasodilators, angiotensin converting enzyme inhibitors, angiotensin receptor antagonists, proliferation and migration inhibitors for smooth muscle cells, platelet aggregate inhibitors, anticoagulants, migration inhibitor of chemical mediators, growth promoters or inhibitors of vascular endothelial cells, aldose reductase inhibitors, mesangial cell proliferation inhibitors, lipoxygenase inhibitors, immune inhibitors, immune activators, antiviral drugs, Maillard reaction inhibitors, amyloidosis inhibitors, nitrogen monoxide synthesis inhibitors, AGEs (advanced glycation endproducts) inhibitors, radical scavengers, peptides, glycosaminoglycan and derivatives thereof, and oligosaccharides, polysaccharides and derivatives thereof.

The anticancer agents include, for example, carboplatin, cisplatin, oxaliplatin, doxorubicin hydrochloride, epirubicin hydrochloride, gemcitabine hydrochloride, irinotecan hydrochloride, topotecan hydrochloride, vinorelbin tartarate, vincristine sulfate, camptotecin, paclitaxel, and docetaxel.

The immune inhibitors include, for example, rapamycin and tacrolimus.

The proteins include, for example, interferon (IFN), L-asparaginase, catalase, uricase, glucoserebrosidase, adenosinedeaminase, hemoglobin, interleukin 2, interleukin 10, granulocyte colony-stimulating factor, macropharge colony-stimulating factor, superoxide dismutase, urokinase, tPA (tissue-type plasminogen activator), erythropoietin, hydrolases, oxidation-reduction enzymes, antibodies and the like.

The physiologically active substance modified with the first hydrophilic polymer is one which consists of such a first hydrophilic polymer and physiologically active substance as set out hereinbefore. The first hydrophilic polymer and the physiologically active substance are not critical with respect to the combination thereof. For the physiologically active substance modified with the first hydrophilic polymer, there can be used those obtained by appropriate combination of a first hydrophilic polymer and a physiologically active substance depending on the purpose. The physiologically active substance modified with a first hydrophilic polymer includes, for example, an interferon preparation modified with polyethylene glycol. The interferon preparation modified with polyethylene glycol is specifically made of a compound wherein gene-recombinant interferon $\alpha$-2a serving as a mother compound is chemically modified with one molecule of a branched polyethylene glycol (PEG) having a molecular weight of 40,000 daltons.

Where PEG is used as the first hydrophilic polymer used for the modification of a physiologically active substance, the molecular weight of PEG is larger than a molecular weight of PEG ordinarily used to modify a liposome and ranges 5000 to 50000 daltons. When a protein is used as the physiologically active substance, it is usual that the molecular weight of PEG, the number of introduced PEG, and the site where PEG is introduced into the protein have been determined so as to improve the retention in blood without impeding the function of the protein. The physiologically active substance modified with the first hydrophilic polymer may be prepared by a conventionally known procedure.

Next, the second hydrophilic polymer is described.

For the second hydrophilic polymer, there may be used ones that are conventionally known as a hydrophilic polymer. For the specified second hydrophilic polymer, mention is made, for example, of those similar to the foregoing first hydrophilic polymers.

In the practice, it is especially important how to select a second hydrophilic polymer. Where a preparation modified with the first hydrophilic polymer is, for example, a liposome, the second hydrophilic polymer used should preferably have a structure capable of competitively inhibiting a phenomenon of the liposome being recognized with a complement activation substance (e.g. immune cells). This is true of the case where a preparation is a protein modified with the first hydrophilic polymer, i.e. the second hydrophilic polymer should preferably have a structure capable of competitively inhibiting a phenomenon where the first hydrophilic polymer bound to the protein is recognized with a complement activation substance.

More particularly, the first hydrophilic polymer and the second hydrophilic polymer should preferably have commonly at least one same or similar unit structure, and more preferably have at least one same unit structure.

The "unit structures are similar between the first hydrophilic polymer and the second hydrophilic polymer" means that the unit structures of both independently have a hydrophilic group and that the carbon atoms in the unit structure of the second hydrophilic polymer are within $\pm 3$ in number of the carbon atoms in the unit structure of the first hydrophilic polymer (provided that the unit structure of the second hydrophilic polymer has one or more of carbon atoms). It will be noted that with regard to the carbon atoms in the unit structure of the second hydrophilic polymer, where the unit structure of the first hydrophilic polymer is made, for example, of $-CH_2CH_2O-$, the carbon atoms in the unit structure of the first hydrophilic polymer are at 2. Accordingly, the carbon atoms in the unit structure of the second hydrophilic polymer are within a range of 1 to 5. The case where the terminal group of the unit structure is an alkoxy group such as a methoxy group or the like is considered as providing a similar structure.

For the hydrophilic group, mention is made, for example, of a carboxy group, a carbonyl group, a hydroxyl group, an amino group, an amide bond, and an ether bond. Of these, a hydroxyl group and an ether bond are preferred.

Preferred carbon atoms in the unit structure of the second hydrophilic polymer are within a range of $\pm 1$ of the carbon atoms in the unit structure of the first hydrophilic polymer.

It is preferred that such same or similar unit structure as set out hereinabove is, for example, at least one member selected from the group consisting of $-(CH_2CH_2O)_n-$, $-(CH_2CH_2CH_2O)_n-$, $-(CH_2CH(OH)CH_2O)_n-$, and $-(CH_2CH(CH_2OH)O)_n-$. In the formulas, n is an integer of 1 or over.

Of these, those of the formulas wherein n is an integer of 1 to 1,000 are preferred.

It will be noted that n in the unit structure of the first hydrophilic polymer and n in the unit structure of the second hydrophilic polymer are, respectively, set independently and may be same.

Where the carrier is made of a liposome, the most general-purpose polymer used as the first hydrophilic polymer is PEG. In this case, it is most preferred to select PEG as the second hydrophilic polymer. If polypropylene glycol or polyglycerine (PG) is used, for example, as the first hydrophilic polymer, it is most preferred that a hydrophilic polymer of the same type as the first hydrophilic polymer is added as a second hydrophilic polymer.

In the practice, the molecular weight and the amount of the second hydrophilic polymer are not critical, respectively. For instance, assuming that a second hydrophilic polymer is added to a diluent, a smaller molecular weight of the second hydrophilic polymer is preferred in view of higher solubility thereof. More particularly, the second hydrophilic polymer should preferably have a molecular weight of not higher than 50000 daltons. If the molecular weight is not higher than 1000 daltons, a second hydrophilic polymer is, in most cases, liquid in nature, with ease in use.

There needs to be a specific attention to unit mols of the first hydrophilic polymer and the second hydrophilic polymer. Accordingly, it is very important how to manage the unit mols.

The "unit mols" is expressed in terms of the molecular weight of a hydrophilic polymer, the molecular weight of a unit structure and the moles of the hydrophilic polymer and can be calculated according to the following equation.

$$\text{Unit mols} = (\text{molecular weight of hydrophilic polymer}) \div (\text{molecular weight of unit structure}) \times (\text{moles of hydrophilic polymer}) \quad \dots (1)$$

In this way, the unit mols are determined depending on the molecular weight and moles of the first hydrophilic polymer or second hydrophilic polymer.

For example, in case where 5 moles of PEG having a molecular weight of 400 daltons are used as a first hydrophilic polymer modifying the surface of a liposome wherein PEG has a molecular weight of one unit structure ($-CH_2CH_2O-$) of 44, the unit mols of PEG is calculated according to the equation (1) as follows:

$$400 \div 44 \times 5 = 45.45$$

If a liposome is modified with PEG, the molecular weight of the PEG is generally at 2000 to 5000 daltons.

Specifically, in view of the retention in the blood of the PEG-modified preparation, a preferred retention in blood can be obtained at smaller moles of PEG when using PEG having a molecular weight of 5000 daltons rather than PEG having a molecular weight of 2000 daltons.

As to the second hydrophilic polymer, a larger molecular weight of the second hydrophilic polymer results in a greater number of unit mols, thus leading to a smaller number of mols of a required second hydrophilic polymer. In contrast, if a molecular weight of the second hydrophilic polymer becomes smaller, the unit mols becomes smaller in number, thus resulting in a greater number of mols of a required second hydrophilic polymer.

In the invention, the ratio between the unit mols of the first hydrophilic polymer and the unit mols of the second hydrophilic polymer (unit mols of first hydrophilic polymer:unit mols of second hydrophilic polymer) is referred to as unit molar ratio.

The unit molar ratio is preferably at 1:0.1 to 1:1000, more preferably 1:0.5 - 1:100, further preferably 1:0.75 to 1:50 and most preferably 1:1 to 1:50 wherein the dosage of the second hydrophilic polymer is equimolar to or over the first hydrophilic polymer.

Although depending on the in vivo dynamic feature, the unit molar ratio is preferably at 1:0.1 to 1:10000, more preferably 1:0.5 to 1:1000, and most preferably 1:0.75 to 1:200.

It will be noted that the "higher unit molar ratio" means that a ratio by unit mol of the second hydrophilic polymer relative to the first hydrophilic polymer is larger.

If PEG is selected as a first hydrophilic polymer, care should be paid to the discrimination of a molecular weight of the PEG to be used after calculation of unit mols. This is because if the unit mols of the first hydrophilic polymer and second hydrophilic polymer are at the same level, the unit molar ratio could be preferably increased so far as the unit mols of the first hydrophilic polymer bound to a carrier or protein can be reduced. Smaller unit mols of the first hydrophilic polymer bound to a carrier or protein relative to the unit mols of the second hydrophilic polymer leads to better results. In this connection, however, if a carrier is made of a closed endoplasmic reticulum such as a liposome, the retention in blood unfavorably lowers unless the liposome is bound to the surface of the hydrophilic polymer.

The amount of the first hydrophilic polymer sufficient to provide such a good retention in blood is at 0.2 to 10 mol% in lipid membrane components. Accordingly, it is preferred that the second hydrophilic polymer has unit mols greater than the unit mols contained in the above-indicated amount of the first hydrophilic polymer.

For a method of controlling a unit molar ratio, mention is made, for example, of a method wherein the amount/molecular weight of a second hydrophilic polymer are increased or decreased, and a method wherein the amount/molecular weight of a first hydrophilic polymer are increased or decreased. In the practice, discrimination has to be made based on the total unit mols of the first hydrophilic polymer and the second hydrophilic polymer, not based on the molecular weight and mols of each of the first hydrophilic polymer and the second hydrophilic polymer.

The present invention makes use of the concept of competitive inhibition, for which the unit mols of the second hydrophilic polymer should preferably be not smaller than or equal to those of the first hydrophilic polymer. In view of the above, a higher unit molar ratio is better, and in practice, such a range as indicated before is proper.

As stated hereinabove, according to the invention, although the unit molar ratio is important, this is directed to a concept of the case of aiming at an effect of competitive inhibition. More particularly, where the second hydrophilic polymer is present in amounts sufficient to ensure stability of an in vivo preparation, or where a xenobiotic recognition mechanism of a preparation modified with the first hydrophilic polymer is masked with the second hydrophilic polymer, consideration should not be given any more to the amount of the first hydrophilic polymer.

Although the dose of the second hydrophilic polymer is not critical, a preferred one is not less than equivalent, in unit molar ratio, to the first hydrophilic polymer bound to a carrier.

The medicinal composition of the invention may further contain medically allowable diluents depending on the administration route. Examples of the diluent include water, physiological saline, medically allowable organic solvents, collagen, polyvinyl alcohol, polyvinylpyrrolidone, carboxyvinyl polymer, sodium carboxymethylcellulose, sodium polyacrylate, sodium alginate, water-soluble dextran, sodium carboxymethylstarch, pectin, methyl cellulose, ethyl cellulose, xanthan gum, gum arabi, casein, gelatin, agar-agar, diglycerine, prolylene glycol, vaseline, paraffin, stearyl alcohol, stearic acid, human serum albumin (HAS), PBS, bioerodible polymers, serum-free media, surface active agents tolerated as medical additives, and in vivo tolerable, physiological pH buffer solutions. Depending on the type of preparation, the diluent used is appropriately selected from those indicated above although not limited thereto. Of these, water and physiological saline are preferred. Such diluents may be used singly or in combination of two or more.

The diluent is preferably contained in amounts of 0.001 to 10 wt% in a medicinal composition.

The preparation modified with the first hydrophilic polymer and the second hydrophilic polymer should be preferably dispersed in such a diluent as indicated above (preferably in water or physiological saline). Where the preparation modified with the first hydrophilic polymer and the second hydrophilic polymer dispersed in a diluent, the second hydrophilic polymer is in a state of being freed from the preparation modified with the first hydrophilic polymer or in a state of contact with the preparation modified with the first hydrophilic polymer.

The medicinal composition of the invention may further include a stabilizer and/or antioxidant that is medically tolerable depending on the administration route. The stabilizer is not critical and mention is made, for example, of glycerol, mannitol, sorbitol, lactose, or saccharides such as sucrose.

For the antioxidant, no critical limitation is placed thereon and mention is made, for example, of ascorbic acid, uric acid, and tocopherol homologs (e.g. vitamin E). It will be noted that tocopherol includes four types of $\alpha$, $\beta$, $\gamma$ and. $\delta$ isomers, all of which are usable in the practice of the invention. The stabilizer and/or antioxidant used is appropriately selected from those mentioned above depending on the type of preparation, but are not limited thereto. The stabilizers and antioxidants may be used singly or in combination of two or more, respectively. From the standpoint of antioxidation, the above dispersion should preferably be packed in nitrogen-filled package.

The medicinal composition of the invention can be prepared by a conventionally known procedure. The medicinal composition of the invention can be stored by an ordinary method, e.g. by cold storage at 0 to 8°C or by storage at room temperature of 1 to 30°C.

The significant feature of the medicinal composition of the invention resides in that only an immunoreaction can be inhibited without impeding retention in blood of a preparation. More particularly, the feature resides in that instead of enabling immune cells to prevent recognition of the first hydrophilic polymer modifying the preparation, some complement activating substance competitively inhibits recognition of the first hydrophilic polymer by the presence of the second hydrophilic polymer and further inhibits reactions (e.g. complement activation, an ABC phenomenon, anaphylactoid/anaphylactic reactions and the like) subsequent to the recognition.

Next, the preparation of the invention is described.

The preparation of the invention is defined in claim 7 and is one, which contains polyethylene glycol having a molecular weight ranging from 400 to 4000 Dalton as an effective ingredient. This is used to inhibit an immunoreaction as would be caused when using a liposome as defined in claim 1 or 2.

The preparation of the invention should preferably contain, aside from the effective ingredient of the second hydrophilic polymer, a diluent that is medically tolerable depending on the route of administration. Such a diluent may be similar in type to a diluent usable in the medicinal composition of the invention, and in particular, water and physiological saline are preferred. The amount of the second hydrophilic polymer preferably ranges 0.001 to 20 wt% of the preparation.

The preparation of the invention may further contain a stabilizer and/or antioxidant that is medically tolerable depending on the route of administration. Specific examples of the stabilizer and antioxidant are same as those indicated hereinbefore.

The preparation of the invention is used to prevent an immunoreaction as may occur when using a commercially available preparation modified with a hydrophilic polymer or a preparation modified with such a first hydrophilic polymer as set out hereinabove (both of which are hereinafter referred to as "modified preparation"). In this connection, the preparation of the invention may be administered simultaneously with a modified preparation, prior to administration of the modified preparation, or sequentially. Of these, it is preferred to administer the preparation of the invention simultaneously with or prior to the administration of a modified preparation.

Next, a combined preparation is illustrated.

The combined preparation is one wherein a preparation using a second hydrophilic polymer as an effective ingredient and a preparation modified with a first hydrophilic polymer are combined together. The second hydrophilic polymer is used to inhibit an immunoreaction as would be caused by the preparation modified with the first hydrophilic polymer.

The preparation using a second hydrophilic polymer as an effective ingredient is same as the preparation of the invention illustrated hereinbefore.

The preparation modified with a first hydrophilic polymer is same as the preparation modified with a first hydrophilic polymer as contained in the medicinal composition of the invention illustrated hereinbefore.

The preparation modified with a first hydrophilic polymer may include a diluent, stabilizer and antioxidant. The preparation modified with a first hydrophilic polymer may take a form of dry powder or freeze dried powder.

In the combined preparation, the ratio between the unit mols of the first hydrophilic polymer and the unit mols of the second hydrophilic polymer is preferably at 1:0.1 to 1:1000, more preferably at 1:0.5 to 1:100 and most preferably at 1:0.75 to 1:50.

Alternatively, depending on the disposition characteristic of a liposome, the unit molar ratio is preferably at 1:0.1 to 1:10000, more preferably at 1:0.5 to 1:1000 and most preferably at 1:0.75 to 1:200.

In the practice, although the unit molar ratio is important, this is a concept in case where an effect of competitive inhibition is intended. More particularly, if the second hydrophilic polymer is present in an amount sufficient to ensure in vivo stability of a preparation, or if the xenobiotic recognition mechanism of the preparation modified with the first hydrophilic polymer is masked by means of the second hydrophilic polymer, no consideration should be given to the amount of the first hydrophilic polymer any more.

The combined preparation is one which is made of a combination of a preparation containing a second hydrophilic polymer as an effective ingredient and a preparation modified with a first hydrophilic polymer, i.e. a set product.

The combined preparation enables the second hydrophilic polymer to inhibit an immunoreaction induced by the preparation modified with a first hydrophilic polymer. Accordingly, the combined preparation can be used in such a way that the preparation containing the second hydrophilic polymer as an effective ingredient is administered simultaneously with the preparation modified with the first hydrophilic polymer, or prior to the administration of the preparation modified with the first hydrophilic polymer, or sequentially. Of these, it is preferred to administer the preparation using the second hydrophilic polymer as an effective component simultaneously with the preparation modified with the first hydrophilic polymer, or prior to the administration of the preparation modified with the first hydrophilic polymer.

The medicinal composition and preparation of the invention (which may be sometimes referred to hereinafter as "medicinal composition and others of the invention" hereinafter) may take the form, for example, of a powder, gel, solution, emulsion or dispersion. It is especially beneficial and simple that a parenteral preparation containing a calculated amount of a preparation along with a desired pharmaceutical vehicle is produced. Administration through parenteral (intravenous, subcutaneous and intramuscular) injections containing a combined preparation of the invention is possible.

As stated hereinabove, the medicinal composition and others of the invention can competitively inhibit, by means of the second hydrophilic polymer, a phenomenon that the first hydrophilic polymer is recognized with a complement activating substance. In other words, activation of a complement system can be suppressed, which has been difficult to achieve according to conventional methods, thereby ensuring excellent stability on use as a medicinal product. Accordingly, the medicinal composition and others of the invention are exposed to a target site over a long time at high concentration, for which they can be parenterally, systemically or locally administered to hosts (patients). For a host that is an object of administration, mention is made, for example, of mammals, preferably human beings, monkey, rat, poultry and the like.

For the routes of parenteral administration of the medicinal composition and others of the invention, mention is made, for example, of intravenous injection (i.v.) such as drip infusion or the like, intramuscular injection, interperitoneal injection, and subcutaneous injection, from which appropriate selection is possible. The manner of administration can be appropriately selected depending on the age and symptoms of a patient.

The drug contained in the medicinal composition and others of the invention is administered to a patient having already suffered from a disease in amounts sufficient to cure the symptoms of the disease or to inhibit at least partly. For instance, an effective amount of administration of a drug included in a carrier is selected within a range of 0.01 mg to 100 mg per kilogram of body weight in a day. In this connection, however, the drug contained in the medicinal composition and others of the invention is not limited to such a dose.

The second hydrophilic polymer used in the medicinal composition and others of the invention is administered in amounts sufficient to inhibit an immunoreaction induced by the recognition of the first hydrophilic polymer with a complement activating substance. More particularly, administration is made such that the unit mols of the second hydrophilic polymer are equal to or greater than the unit mols of the first hydrophilic polymer.

In the practice, although the unit molar ratio is important, this is a concept in case where an effect of competitive inhibition is intended. More particularly, if the second hydrophilic polymer is present in an amount sufficient to ensure in vivo stability of a preparation, or if the xenobiotic recognition mechanism of the preparation modified with the first hydrophilic polymer is masked by means of the second hydrophilic polymer, no consideration should be given to the amount of the first hydrophilic polymer any more. However, no limitation is placed on these doses with respect to the medicinal composition and others of the invention.

With respect to the administration timing, the medicinal composition and others of the invention may be administered after the occurrence of a disease, or may be preventively administered so as to relieve symptoms in onset at the time when the onset of a disease is assumed. The administration time can be appropriately selected depending on the age and symptoms of a patient.

The specific manner of administration of the medicinal composition and others of the invention includes, for example, an administration method using a syringe or drip infusion, and a method wherein a catheter is inserted into the body (e.g. the lumen or blood vessel) of a patient or host, and its tip is lead to the vicinity of a target site, under which administration is made through the catheter from a desired target site or the vicinity thereof, or a site where a blood stream to the target site is expected.

Examples

The invention is illustrated in more detail by way of examples and should not be construed as limited to these examples and test examples.

The respective concentrations and particle sizes of drug-entrapping liposomes prepared in examples were determined in the following ways.

- Phospholipid concentration (mg/mL): a phospholipid (HSPC) concentration in a liposome dispersion quantitatively determined by use of a quantitative kit of phospholipid (Phospholipid C Test Wako, made by Wako Pure Chemical Industries, Ltd.)
- Total lipid concentration (mol/L): a total molar concentration of surface modifier-containing membrane components calculated from the above phospholipid concentration.
- Particle size (nm): an average particle size determined by diluting 20 μL of a liposome dispersion diluted with physiological saline to make 3 mL, followed by measurement with Zetasizer 3000HS (made by Malvern Instruments).

• Preparation of a plasma after administration of a PEG-modified liposome

In the practice of the invention, for a testing subject in the following test examples 1 to 4, a plasma obtained after administration of a PEG-modified liposome to a dog plasma (i.e. a dog plasma wherein an immunoreaction is enhanced) was used for carrying out the experiment. Up to now, it has been reported that if a PEG-modified liposome is administered to a living body (e.g. a dog) plural times, there occur anaphylactoid/anaphylactic reactions involving complement activation. Accordingly, it is considered that the plasma of a living body that has been once administered with a PEG-modified liposome is immunoenhanced against the PEG-modified liposome. For this reason, there was used, for testing, the plasma (derived from a dog) obtained after administration of the PEG-modified liposome in order to seek for a measure wherein the PEG-modified liposome escaped from an immune system.

The plasma after administration of the PEG-modified liposome was obtained in the following manner. Initially, the PEG-modified liposome (6 pmols/kg calculated as a total amount of lipids) obtained in the following Preparatory Example 1 was administered to a dog and blood was obtained by collection after 7 days. The blood was subjected to centrifugal separation to obtain a plasma. The plasma prepared in this way is hereinafter referred to as "liposome-treated plasma".

The abbreviations and molecular weights of the respective ingredients used are indicated below.

- HSPC: hydrogenated soybean phosphatidylcholine (with a molecular weight of 790, made by Lipoid Co.)
- Chol: cholesterol (with a molecular weight of 386.65 made by Solvay Co.)
- $PEG_{5000}$-PE: polyethylene glycol (with a molecular weight of 5000)-phosphatidylethanolanine (with a molecular weight of 5938, made by Genzyme Corp.)
- DOX: doxorubicin hydrochloride (molecular weight: 579.99)
- $PEG_{400}$: polyethylene glycol: (molecular weight: 400)
- $PEG_{4000}$: polyethylene glycol: (molecular weight: 4000)
- $PEG_{20000}$: polyethylene glycol: (molecular weight: 20000)
- $PPG_{425}$: polyethylene glycol: (molecular weight: 425)
- PSB: physiological saline-phosphate buffer solution (with a pH of 7.2)

Preparatory Example 1

(1) Preparation of a liposome

0.706 g of hydrogenated soybean phosphatidylcholine (HSPC) and 0.294 g of cholesterol (Chol) were added to a anhydrated ethanol (1 mL, made by Pure Chemical Industries, Ltd.)-PBS (pH 7.2, 9 mL) solution heated to 60°C and swollen satisfactorily, and were agitated by means of a Vortex mixer, followed by successively passing through filters (pore size of 0.2 μm × 5 times and 0.1 μm × 10 times, made by Whatman Co.) attached to an extruder (The Extruder T.100, Lipex Biomembranes Inc.) at 68°C to prepare a liposome dispersion.

(2) Introduction of $PEG_{5000}$-PE

2.0 mL (corresponding to 0.75 mol% of a total lipid amount of mixed lipids) of an aqueous solution (36.74 mg/mL) of $PEG_{5000}$ (which corresponds to a first hydrophilic polymer)-phosphatidylethanolamine (which may be hereinafter referred to sometimes as "$PEG_{5000}$-PE") diluted with distilled water, followed by heating at 60°C for 30 minutes and agitation. In this way, the $PEG_{5000}$-PE was introduced into the liposome to obtain a dispersion of a $PEG_{5000}$-modified liposome (which may be hereinafter referred sometimes as "$PEG_{5000}$-modified liposome").

(3) Purification step

The dispersion of the $PEG_{5000}$-modified liposome was purified by use of a gel column substituted with PBS (pH 7.2). After gel filtration, the resulting sample was ice cooled. Next, using high-performance liquid chromatography, the membrane composition of HSPC, Chol and $PEG_{5000}$-PE contained in the $PEG_{5000}$-modified liposome and lipid concentrations were quantitatively determined. The results are shown in Table 1.

The unit mols (unit mols/mL) of PEG in the liposome are calculated according to the following equation (1). The molecular weight of $PEG_{5000}$ (first hydrophilic polymer)-PE was taken as 6075 and the number of units contained per mole of $PEG_{5000}$ is taken as 113.6.

Unit mols of PEG (unit mols/mL) = (lipid concentration of $PEG_{5000}$-PE used, mg/mL) ÷ 1000 ÷ 6075 × 113.6 ...(1)

The $PEG_{5000}$-modified liposome obtained above had a unit mol concentration (unit mols/mL) of $35.0 \times 10^{-6}$. The particle size of the resulting $PEG_{5000}$-modified liposome was found to be at 95.3 nm.

[Table 1] Table 1 (liposome modified with the first hydrophilic polymer $PEG_{5000}$)

| Preparatory Example | Membrane composition (molar ratio) | Lipid concentration (mg/mL) | | |
|---|---|---|---|---|
| | HSPC/Chol/$PEG_{5000}$-PE | HSPC | Chol | $PEG_{5000}$-PE |
| | 53.60/45.65/0.75 | 18.67 | 7.60 | 1.87 |

Preparatory Example 2

Second hydrophilic polymers indicated in Table 2 were, respectively, added 10 ml of the dispersion of the $PEG_{5000}$ (first hydrophilic polymer)-modified liposome prepared in Preparatory Example 1 to provide preparations. The second hydrophilic polymers used are indicated in Table 2. It will be noted that the amounts of the respective second hydrophilic polymers were calculated based on the results obtained in Test Examples 1 to 3 described hereinafter. The unit molar ratio is a ratio of the unit moles of each second hydrophilic polymer to the unit mols of the first hydrophilic polymer ($PEG_{5000}$) bound to the $PEG_{5000}$ (first hydrophilic polymer)-modified liposome prepared in Preparatory Example 1.

[Table 2]

Table 2 (amounts of the second hydrophilic polymers in 10 mL of preparation)

| | Second hydrophilic polymer (molecular weight) | Molecular weight of one unit structure | Unit mols of one strand | Unit mols of the first hydrophilic polymer in 10 mL ($\times 10^{-5}$ unit mols) | Unit mols of the second hydrophilic polymer in 10 mL ($\times 10^{-5}$ unit mols) | Unit molar ratio |
|---|---|---|---|---|---|---|
| Preparatory Example 2 | PEG (400) | 44 | 9.1 | 35.0 | 113.6 | 3.2 |
| | PPG (425) * | 58 | 7.3 | 35.0 | 86.1 | 2.4 |
| * reference example | | | | | | |

The unit mols of the first hydrophilic polymer (PEG) in 10 mL in Table 2 were calculated according to the following equation (2). From the results of Table 1, calculation was made such that the lipid concentration of the employed $PEG_{5000}$-PE was at 1.87 mg/mL, the molecular weight of the $PEG_{5000}$ (first hydrophilic polymer)-PE was at 6075, and the units contained per mol of $PEG_{5000}$ was at 113.6.

Unit mols of the first hydrophilic polymer (PEG) in

10 mL =

1.87 (mg/mL) ÷ 1000 × 100 (mL) ÷ 6075 × 113.6 ...(2)

The unit mols of the second hydrophilic polymer (PEG) in 10 mL in Table 2 were calculated according to the following equation (3).

Unit mols of the second hydrophilic polymer (PEG) in

10 mL = [value obtained in equation (2)] × 3.2 (unit molar

ratio) ...(3)

The unit molar ratio was calculated from the concentration of the second hydrophilic polymer used in Test Examples 1 to 3 being at 100 pg/mL.

Test Example 1 Evaluation of complement activation

First, 2 $\mu$L of PEG's (with molecular weights of 400, 4000 and 20000 at a final concentration of 100 pg/mL) serving as a second hydrophilic polymer was added to 400 $\mu$L of the liposome-treated plasma, followed by keeping at 37°C for 10 minutes. Thereafter, 3 $\mu$L of physiological saline or PEG-modified liposome was added to 150 $\mu$L of each plasma pretreated with PEG as set out above and reacted while keeping at 37°C for 30 minutes.

• Measurement of a complement value (CH50)

The respective plasmas pretreated with three types of PEG's were divided and placed in two containers each in an amount of 150 $\mu$L, followed by adding 3 $\mu$L of physiological saline or the $PEG_{5000}$-modified liposome to each container for reaction. After completion of the reaction, the complement values (CH50) of the respective reaction solutions were measured by use of a commercially available measuring kit. The commercially available measuring kit is to determined a complement value based on the 50% hemolysis procedure, which has been established by Mayer et al as a measuring method of a complement. One unit of a complement in this method means a quantity necessary for hemolyzing 50% of $5 \times 10^8$ sheet erythrocytes sensitized with an optimum concentration of a hemolysin in a reaction amount of 7.5 mL at 37°C for 60 minutes.

• Complement activation rate

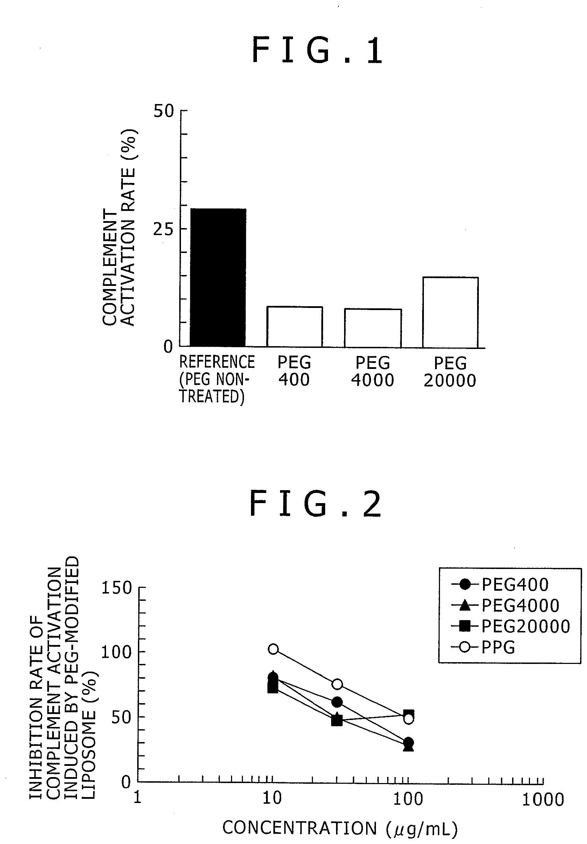

The complement value (CH50) measured in a manner as set forth above is applied to the following equation (4) to obtain a percentage complement activation. The results are shown in Fig. 1.

Complement activation rate (%) = [1 – (CH50 at the

time of addition of PEG-modified liposome) ÷ (CH50 at the

time of addition of physiological saline)] × 100 ...(4)

It will be noted that the data of "reference (PEG non-treated)" in Fig. 1 is obtained as follows. 400 $\mu$L of the liposome-treated plasma was pretreated by adding 2 $\mu$L of physiological saline in place of PEG serving as a second hydrophilic polymer. The plasma pretreated with the physiological saline was divided and placed in two containers each in an amount of 150 pL, followed by addition of 3 $\mu$L of physiological saline or $PEG_{5000}$-modified liposome to the respective containers and reaction while keeping at 37°C for 30 minutes. After the reaction, the complement values (CH50) in the respective reaction solutions was measured by use of a commercially available measuring kit in the same manner as set out hereinabove. The resulting complement values (CH50) were applied to the equation (4) to obtain a percentage complement activation.

In Fig. 1, lower values in the bar graphs lead to lower degrees of complement activation, indicating that recognition of the PEG$_{5000}$-modified liposome with a complement activating substance (an antibody, a lectin or the like) is suppressed with a small risk of causing the anaphylactoid/anaphylactic reactions to occur. As will be apparent from Fig. 1, activation of the complement system by the action of the PEG$_{5000}$-modified liposome can be inhibited by the pretreatment of PEG. It was confirmed that the inhibition action becomes strongest when PEG has a molecular weight of 400 to 4000. In view of the past record of intravenous injection of PEG$_{400}$, the concentration (100 $\mu$g/mL) is 1/5 of that injection, thus presenting no problem when administration is given to human beings. On the other hand, PEG$_{4000}$ is about 2.5 times as great as the track record of intravenous injection, so that it is necessary to collect data on stability again. The foregoing results suggest that pre-administration of PEG is effective for keeping low the activation of a complement system with the PEG-modified preparation and the risk for subsequent anaphylactoid/anaphylactic reactions. More particularly, it has been confirmed that the pre-administration of PEG can inhibit the complement activation caused by the PEG-modified preparation and the anaphylactoid/anaphylactic reactions.

Test Example 2 Evaluation on inhibition of complement activation with a hydrophilic polymer

Complement activation inhibiting effects were checked by comparison using, aside from PEG$_{400}$, PEG having a different molecular weight and PPG having substantially the same molecular weight but with a different structure as a second hydrophilic polymer used for inhibiting complement activation caused by a liposome modified with a first hydrophilic polymer.

After addition of 2 $\mu$L (at a final concentration of 10 to 100 pg/mL) of PEG$_{400}$, PEG$_{20000}$ or PPG, used as a second hydrophilic polymer, to 400 $\mu$L of a liposome-treated plasma, the resulting solution was kept at 37°C for 10 minutes. For reference, (non-treated second hydrophilic polymer), those pretreated with 2 $\mu$L of physiological saline were provided. Next, the respective plasmas pretreated with such second hydrophilic polymers were divided and placed in two containers each in an amount of 150 $\mu$L. 3 $\mu$L of physiological saline or a PEG$_{5000}$(first hydrophilic polymer)-modified liposome was added to each of the two containers for individual plasmas, followed by reaction while keeping at 37°C for 30 minutes. Next, the complement values (CH50) of the respective reaction solutions were measured in the same manner as described above and the percentage complement activation of the respective reaction solutions was determined according to the foregoing formula (4). The inhibition rate against complement activation of the PEG$_{5000}$-modified liposome through the pretreatment with various types of second hydrophilic polymers were determined according to the following equation (5). The results are shown in Fig.2.

$$\text{Complement activation inhibiting rate (\%)}$$

$$\text{(percentage complement activation at the time of}$$

$$\text{pretreatment of each second hydrophilic polymer)}$$

$$\div \text{(percentage complement} \cdot \text{activation at the time of}$$

$$\text{pretreatment with physiological saline)} \times 100 \ldots (5)$$

As will be apparent from Fig. 2, the inhibition effect of the complement activation was concentration-dependently confirmed even for PPG whose carbon atoms in the unit structure constituting the main chain are larger by one than PEG. The above results suggest that PEG is structurally preferred. The reason for this is considered as follows: since PEG modifying a liposome and serving as the first hydrophilic polymer and PEG serving as the second hydrophilic polymer have the same unit structure, PEG serving as the second hydrophilic polymer competitively inhibits a complement activating substance from binding to the PEG-modified liposome, thereby leading to more suppressed activation of the complement system.

Test Example 3

The results of Test Example 2 have revealed that when the PEG$_{400}$ used as the second hydrophilic polymer is pretreated, activation of the complement system can be inhibited. In order to elucidate whether or the pretreatment is essential for the second hydrophilic polymer, a difference in activity between the pretreatment and the simultaneous addition of the second hydrophilic polymer (PEG$_{400}$) was checked.

More particularly, with the pretreatment, the second hydrophilic polymer (PEG$_{400}$) was added to the liposome treated plasma at a final concentration of 10 to 100 μg/mL and kept at 37°C for 10 minutes. Next, the plasma pretreated with the second hydrophilic polymer (PEG$_{400}$) and a PEG$_{5000}$(first hydrophilic polymer) modified liposome were mixed at a ratio of 50:1 and reacted while keeping at 37°C for 30 minutes. In the same manner as set out hereinabove, CH50 after the reaction was measured and applied to the equation (5) to obtain a complement activation inhibiting rate.

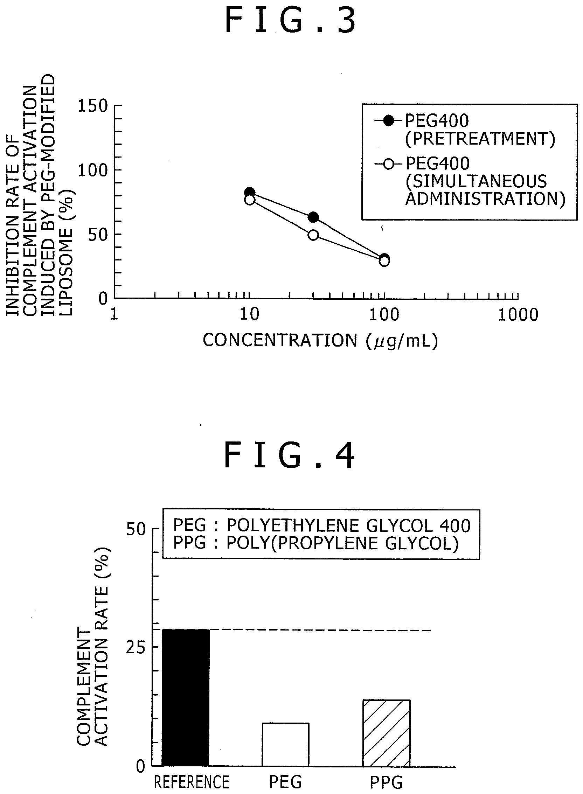

On the other hand, with the case of simultaneous addition, a liposome-treated plasma was kept at 37°C for 30 minutes and reacted, after which PEG$_{400}$ serving as a second hydrophilic polymer and a PEG$_{5000}$ (first hydrophilic polymer) modified liposome were added to and mixed with the plasma, and reacted while keeping at 37°C for 30 minutes. A second hydrophilic polymer (PEG$_{400}$) was added to the liposome-treated plasma to make a final concentration of 10 to 100 pg/mL. A second hydrophilic polymer (PEG$_{400}$ treated plasma and a PEG$_{5000}$ (first hydrophilic polymer)-modified liposome were added at a ratio of 50:1. After reaction, CH50 was measured in the same manner as set out hereinabove and applied to the equation (5) to obtain a complement activation inhibiting rate. The results are shown in Fig. 3.

As will be apparent from Fig. 3, no difference was found between the pretreatment and the simultaneous addition of the second hydrophilic polymer (PEG$_{400}$) with respect to the inhibiting effect of the complement activation. Thus, it was made clear that the inhibiting effect of the second hydrophilic polymer (PEG$_{400}$) on the activation of the complement system with the PEG$_{5000}$ (first hydrophilic polymer) modified liposome could be similarly expected when using either of the pretreatment or the simultaneous addition.

Accordingly, the second hydrophilic polymer for inhibiting complement activation not only can inhibit activation of a complement system through pre-administration, but also can inhibit complement activation and anaphylactoid/anaphylactic reactions by addition to a preparation modified with a first hydrophilic polymer.

Test Example 4

A liposome-treated plasma and the preparation prepared in Preparatory Example 2 [a mixed solution of a PEG$_{5000}$ (first hydrophilic polymer) modified liposome and a second hydrophilic polymer (PEG$_{400}$ or PPG$_{425}$)] were mixed at a ratio of 1:50 and reacted while keeping at 37°C for 30 minutes. In the same manner as set out hereinbefore, a complement value (CH50) was measured and the resulting complement value was applied to the equation (4) to obtain a percentage complement activation.

As will be apparent from Fig. 4, like the results of checking of the pretreatment, simultaneous addition of the second hydrophilic polymer (PEG$_{400}$ or PPG$_{425}$) and a PEG$_{5000}$ (first hydrophilic polymer)-modified liposome lead to the inhibition of activation of the complement system.

As will be apparent from the results of Test Examples 3 and 4, the second hydrophilic polymer can be used separately from or simultaneously with the preparation modified with the first hydrophilic polymer.

Test Example 5: Accelerated stability test for preparations (drug release rate)

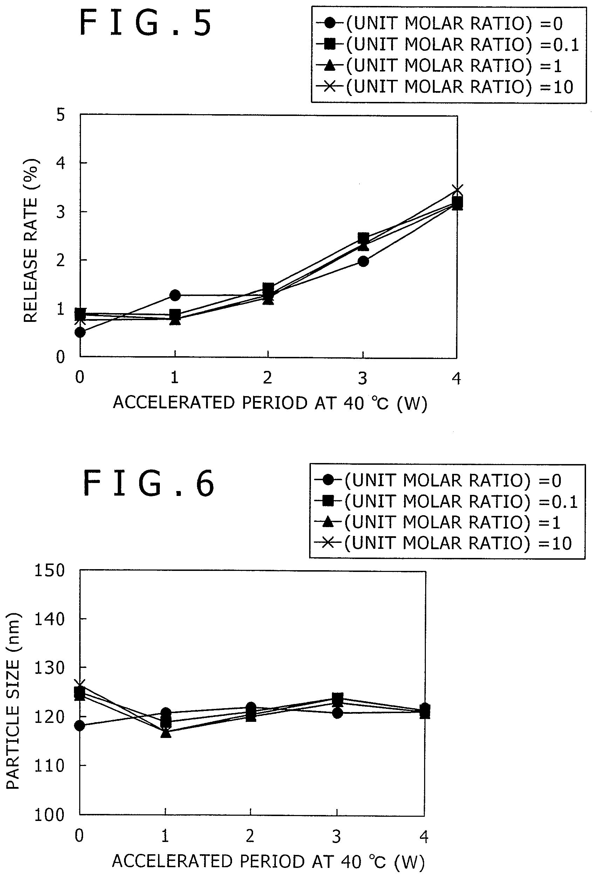

The preparations prepared in the following Preparatory Example 3 were, respectively, dispensed into a 3 mL vial in an amount of 1 mL. The thus dispensed vials were heated to 40°C for 0 to 4 weeks to check a change in the liposome particle size and a change in drug release rate in relation to time. 150 μL of each preparation was centrifugally settled in every week to measure an amount of a drug present prior to ultracentrifugation and an amount of the drug present in an supernatant liquid after the ultracentrifugation by use of spectrophotofluorometer RF-5000 (Shimadzu Corporation). The drug release rate was calculated according to the following equation (6). The results are shown in the graph of Fig. 5. It will be noted that the unit molar ratio in Fig. 5 is expressed by (unit mols of PEG$_{400}$ serving as a second hydrophilic polymer) ÷ (unit mols of PEG$_{5000}$ serving as a first hydrophilic polymer).

```
Drug release rate (%) = (amount of a drug present in

a supernatant liquid after ultracentrifugation - amount of

a drug present prior to ultracentrifugation) ÷ (weight of

total drugs supported in a drug carrier used) × 100 ...(6)
```

As will be apparent from Fig. 5, stable and low drug release rates are shown for all the preparations. No change in the drug release property was found in the presence or absence of the second hydrophilic polymer (PEG$_{400}$) in the preparations. Moreover, no difference in the drug release property was observed depending on the concentration of the second hydrophilic polymer (PEG$_{400}$). In this way, it was not recognized that the presence of the second hydrophilic

polymer (PEG$_{400}$) allowed the liposome modified with the first hydrophilic polymer to be instabilized or the preparation to be lowered in stability.

Test Example 6: Accelerated stability test of preparations (particle size)

The respective preparations prepared in the following Preparatory Example 3 were heated to 40°C for 0 to 4 weeks to check a change in particle size of the liposome modified with a first hydrophilic polymer. 20 μL of a dispersion of the liposome modified with the hydrophilic polymer was diluted to 3 ml with physiological saline. This dilution was analyzed by use of Zetasizer 3000HS (made by Malvern Instruments) in every week to measure the particle size (average particle size) of the liposome. The results are shown in the graph of Fig. 6. It will be noted that the unit molar ratio in Fig. 6 is expressed by (unit mols of PEG$_{400}$ serving as a second hydrophilic polymer) ÷ (unit mols of PEG$_{5000}$ serving as first hydrophilic polymer).

As will be apparent from Fig. 6, where the second hydrophilic polymer (PEG$_{400}$) is present or absent in the diluent, there was no change in the particle size of the liposome modified with the first hydrophilic polymer. No change in the particle size was also found in relation to the concentration of the second hydrophilic polymer (PEG$_{400}$). In this way, it was not recognized that the presence of the second hydrophilic polymer (PEG$_{400}$) allowed the liposome modified with the first hydrophilic polymer or the preparation to be lowered in stability.

Preparatory Example 3: After-introduction method

(1) Preparation of a liposome

0.706 g of hydrogenated soybean phosphatidylcholine (HSPC) and 0.294 g of cholesterol (Chol) were added to 9 mL of a 250 mM ammonium sulfate/water-free ethanol (1 mL, made by Pure Chemical Industries, Ltd.) solution heated to 60°C and swollen satisfactorily, and were agitated by means of a Vortex mixer, followed by successively passing through filters (pore size of 0.2 μm × 5 times and 0.1 μm × 10 times, made by Whatman Co.) attached to an extruder (The Extruder T.10, Lipex Biomembranes Inc.) at 68°C to prepare a liposome dispersion.

(2) Introduction of PEG$_{5000}$-PE

2.0 mL (corresponding to 0.75 mol% of a total lipid amount of mixed lipids) of a distilled water solution (36.74 mg/mL) of PEG$_{5000}$ (a first hydrophilic polymer)-phosphatidylethanolamine (PEG$_{5000}$-PE), followed by heating at 68°C for 30 minutes. In this way, the lipid derivative of polyethylene glycol (first hydrophilic polymer) was introduced into the liposome to obtain a dispersion of a PEG$_{5000}$-modified liposome.

(3) External substitution

The thus obtained liposome was subjected to a gel column substituted with a 10 mM Tris/10% sucrose solution (pH 9.0) for external aqueous phase substitution.

After the external aqueous phase substitution, the resulting liposome was subjected to quantitative determination of a HSPC concentration by use of a phospholipid quantitative determination kit. The total lipid amount mM was obtained from the HSPC concentration.

(4) Entrapment of a drug

A 10 mg/mL doxorubicin (DOX) hydrochloride-10 mM Tris/10% sucrose solution (pH 9.0) was prepared.

This doxorubicin hydrochloride solution was added to a PEG$_{5000}$-modified liposome dispersion in such an amount as to make DOX/total lipids = 0.2 (mol/mol), followed by agitation at 60°C for 60 minutes thereby introducing doxorubicin hydrochloride. The sample obtained after the introducing was ice cooled. The liposome dispersion after entrapping of the doxorubicin hydrochloride was subjected to a gel column substituted with a 10 mM histidine-10% sucrose solution (pH 6.5) for gel filtration, thereby removing an unentrapped drug therefrom.

Next, the DOX preparation obtained above was diluted with a 10 mM histidine-10% sucrose solution (pH 6.5) and adjusted in total lipid concentration to 15 mM.

Next, the unit moles of the bound PEG$_{5000}$ (first hydrophilic polymer) in 5 mL of the DOX preparation were calculated, and PEG$_{400}$ serving as a second hydrophilic polymer was added in such amounts that unit moles of the PEG$_{400}$ serving as a second hydrophilic polymer ÷ unit mols of PEG$_{400}$ bound to the DOX preparation and serving as a first hydrophilic polymer = 0, 0.1, 1.0 and 10. The unit mols of the first hydrophilic polymer in 5 mL of the preparation having a total lipid amount of 15 mM was calculated according to the following equation (7). The composition and particle size are shown in Table 3.

$$\text{Unit mols of the first hydrophilic polymer in 5 mL}$$

$$\text{of the preparation whose total lipid amount is at 15 mM}$$

$$= 15 \div 1000 \div 1000 \times 5 \times 0.75 \div 100.75 \times 113.6$$

$$= 63 \times 10^{-6} \text{ (unit mols) } ...(7)$$

[Table 3]

| Preparatory Example | Membrane composition (mol ratio) | | Unit mols of PEG in 5 mL (x $10^{-6}$) | | Unit molar ratio | Lipid concen-tration | Particle size |
|---|---|---|---|---|---|---|---|
| | Lipids | PEG$_{5000}$-PE | PEG$_{400}$ serving as a second hydrophilic polymer | PEG$_{5000}$ serving as a first hydrophilic polymer | PEG$_{400}$/ PEG$_{5000}$ | mM | nm |
| | HSPC/Chol | | | | | | |
| Preparatory Example 3 | 54/46 | 0.75 | 0 | 63 | 0 | 15 | 118.0 |
| | | | 6.3 | 63 | 0.1 | 15 | 124.9 |
| | | | 63 | 63 | 1 | 15 | 125.1 |
| | | | 630 | 63 | 10 | 15 | 126.8 |

Test Example 7

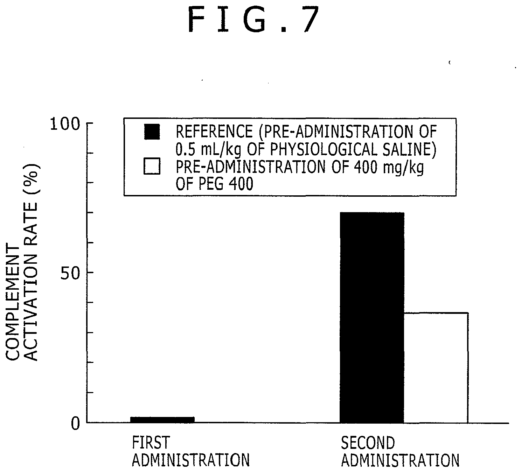

There was checked an effect of PEG$_{400}$ (40 mg/kg) on complement activation and anaphylactoid/anaphylactic symptoms which occurred upon repeated administration, to a dog (beagle, male, 18-19 months in age, body weight: 10.8 - 15.0 Kg), of the PEG-modified liposome (total lipid amount: 6 $\mu$mols/kg) prepared in Preparatory Example 1. The PEG-modified liposome in the form of the stock solution prepared and PEG$_{400}$ in the form of a dilution with physiological saline were intravenously administered from the cephalic vein. More particularly, at the time when the PEG-modified liposome was repeatedly (at intervals of one week) administered in an amount of 6 $\mu$mols/0.17 mL/kg, a group (reference) wherein PEG$_{400}$ was pre-administered in an amount of 40 mg/0.5 mL/kg prior to administration of the PEG-modified liposome and a group wherein 0.5 mL/kg of physiological saline was administered were compared with respect to the complement activation and anaphylactoid/anaphylactic symptoms. It will be noted that the unit molar ratio is such that (unit mols of PEG$_{400}$ serving as second hydrophilic polymer) $\div$ (unit mols of PEG$_{5000}$ serving as first hydrophilic polymer) = 178. With respect to the complement activation, blood was sampled prior to administration of the PEG-modified liposome and 10 minutes after the administration, after which a complement value (CH50) in the plasma was measured to obtain a percentage complement activation. The results are shown in Fig. 7. Fig. 7 is a graph showing the results of a percentage complement activation when PEG$_{400}$ is pre-administered upon repeated administration, to dogs, of the liposome prepared in Preparatory Example 1.

$$\text{Percentage complement activation (\%) } = [1 - (\text{CH50}$$

$$\text{prior to administration of PEG-modified liposome) } \div (\text{CH50}$$

$$\text{after administration of PEG-modified liposome)] } \times 100 \quad (8)$$

As will be apparent from the results shown in Fig. 7, no complement activation was recognized at the initial time of administration of the PEG-modified liposome to the dog for the pre-administration groups of physiological saline and $PEG_{400}$. At the second time of administration, a complement activation of 68.5% was recognized for the pre-administration group of physiological saline, with the $PEG_{400}$ administration group being at 36.0%. Thus, it was made clear that the complement activation at the time of repeated administration of the PEG-modified liposome was suppressed to about 50% through pre-administration of 40 mg/kg of $PEG_{400}$.

Next, the results on the anaphylactoid/anaphylactic reactions are shown in Table 4.

[Table 4]

Table 4

| | Administration group No. | First administration | Second administration |
|---|---|---|---|
| Reference (pre-administration of 0.5 mL of physiological saline + administration of PEG-modified liposome) | 101 | - | Red flare, defecation, difficulty in standing |
| | 102 | - | Incontinence, collapse |
| Pre-administration of 40 mg/kg of PEG-400 + administration of PEG-modified liposome) | 201 | - | - |
| | 202 | - | Red flare |

As will be apparent from the results shown in Table 4, no development of anaphylactoid/anaphylactic symptoms was recognized for both physiological saline and pre-administration of $PEG_{400}$ at the initial administration of the PEG-modified liposome. At the second time of administration, anaphylactoid/anaphylactic symptoms such as red flare, incontinence, defecation, collapse, difficulty in standing and the like were recognized, and only red flare was recognized for the $PEG_{400}$ pre-administration group.

From the forgoing results, it was suggested that pretreatment with low molecular weight PEG was effective for keeping low the risk of the activation of a complement system with the PEG preparation and subsequent anaphylactoid/anaphylactic reactions.

Brief Description of Drawings

[Fig. 1] is a graph showing an inhibiting effect on the complement activation induced with a liposome modified with a first hydrophilic polymer ($PEG_{5000}$) in a plasma treated with the liposome prepared in Preparatory Example 1.

[Fig. 2] is a graph showing for comparison inhibiting actions of second hydrophilic polymers (PEG, PPG) on the complement activation induced with a liposome modified with a first hydrophilic polymer ($PEG_{5000}$) in a plasma treated with the liposome prepared in Preparatory Example 1.

[Fig. 3] is a graph showing for comparison an inhibiting effect of pretreatment and simultaneous treatment with a second hydrophilic polymer (PEG) on the complement activation induced with a liposome modified with a first hydrophilic polymer ($PEG_{5000}$) in a plasma treated with the liposome prepared in Preparatory Example 1.

[Fig. 4] is a graph showing for comparison the complement activity of various types of preparations by use of a plasma treated with the liposome prepared in Preparatory Example 1.

[Fig. 5] is a graph showing the results (release rate) of an accelerated stability test at 40°C for a DOX preparation prepared in Preparatory Example 3.

[Fig. 6] is a graph showing the results (particle size) of an accelerated stability test, at 40°C, of a DOX preparation prepared in Preparatory Example 3.

[Fig. 7] is a graph showing the results of a complement activation rate when $PEG_{400}$ is pre-administered in the course of repeated administration, to a dog, of a liposome prepared in Preparatory Example 1.

Claims

1.   A medicinal composition, wherein the medical composition comprises

a liposome made of hydrogenated phosphatidyl choline and cholesterol and whose surface has been modified with a polyethylene glycol derivative, wherein the polyethylene glycol derivative is polyethylene glycol-phosphatidylethanol amine, and has a molecular weight of the polyethylene glycol of the polyethylene glycol derivative of 5000 dalton, and

a polyethylene glycol, wherein the molecular weight of the polyethylene glycol is in the range from 400 to 4000 dalton.

**2.** The medicinal composition according to claim 1, wherein the medical composition comprises

a liposome made of hydrogenated soybean phosphatidyl choline and cholesterol, and whose surface has been modified with polyethylene glycol-phosphatidylethanol amine, wherein the molecular weight of the polyethylene glycol of the polyethylene glycol-phosphatidylethanol amine is 5000 dalton, and
a polyethylene glycol having a molecular weight of 400 dalton.

**3.** The medicinal composition according to claim 1 or 2 for use in inhibiting anaphylactoid/anaphylactic reactions.

**4.** The medicinal composition according to any one of claims 1 to 3, wherein said liposome whose surface has been modified with said polyethylene glycol derivative and further said polyethylene glycol are dispersed in a diluent.

**5.** The medicinal composition according to any one of claims 1 to 4, wherein said liposome is a drug carrier supporting a drug in a carrier whose surface has been modified with said polyethylene glycol derivative.

**6.** The medicinal composition according to any one of claims 1 to 5, wherein said liposome and said polyethylene glycol are dispersed in water or physiological saline, respectively.

**7.** A preparation for use in inhibiting anaphylactoid/anaphylactic reactions induced by the liposome as defined in claim 1 or 2, wherein the preparation comprises polyethylene glycol, wherein the molecular weight of the polyethylene glycol is in the range from 400 to 4000 dalton.

**8.** The preparation according to claim 7, wherein said polyethylene glycol is dispersed in water or physiological saline.


**Patentansprüche**

**1.** Medizinische Zusammensetzung, wobei die medizinische Zusammensetzung umfasst

ein Liposom hergestellt aus hydriertem Phosphatidylcholin und Cholesterin und dessen Oberfläche modifiziert wurde mit einem Polyethylenglycolderivat, wobei das Polyethylenglycolderivat Polyethylenglycolphosphatidylethanolamin ist und ein Molekulargewicht des Polyethylenglycols des Polyethylenglycolderivats von 5000 Dalton aufweist, und
ein Polyethylenglycol, wobei das Molekulargewicht des Polyethylenglycols in dem Bereich von 400 bis 4000 Dalton ist.

**2.** Medizinische Zusammensetzung nach Anspruch 1, wobei die medizinische Zusammensetzung umfasst

ein Liposom hergestellt aus hydriertem Sojabohnenphosphatidylcholin und Cholesterin, und dessen Oberfläche modifiziert wurde mit Polyethylenglycolphosphatidylethanolamin, wobei das Molekulargewicht des Polyethylenglycols des Polyethylenglycolphosphatidylethanolamins 50000 Dalton ist, und
ein Polyethylenglycol mit einem Molekulargewicht von 400 Dalton.

**3.** Medizinische Zusammensetzung nach Anspruch 1 oder 2 zur Verwendung bei der Hemmung anaphylaktoider/anaphylaktischer Reaktionen.

**4.** Medizinische Zusammensetzung nach einem der Ansprüche 1 bis 3, wobei das Liposom dessen Oberfläche mit dem Polyethylenglycolderivat modifiziert wurde und des Weiteren das Polyethylenglycol in einem Verdünnungsmittel dispergiert sind.

**5.** Medizinische Zusammensetzung nach einem der Ansprüche 1 bis 4, wobei das Liposom ist ein Wirkstoffträger unterstützend einen Wirkstoff in einem Träger dessen Oberfläche mit dem Polyethylenglycolderivat modifiziert wurde.

**6.** Medizinische Zusammensetzung nach einem der Ansprüche 1 bis 5, wobei das Liposom und das Polyethylenglycol jeweils in Wasser oder physiologischer Kochsalzlösung dispergiert sind.

**7.** Herstellung zur Verwendung bei der Hemmung anaphylaktoider/anaphylaktischer Reaktionen verursacht durch das Liposom wie in Anspruch 1 oder 2 definiert, wobei die Herstellung Polyethylenglycol umfasst, wobei das Molekulargewicht des Polyethylenglycols in dem Bereich von 400 bis 4000 Dalton ist.

**8.** Herstellung nach Anspruch 7, wobei das Polyethylenglycol in Wasser oder physiologischer Kochsalzlösung dispergiert ist.

**Revendications**

**1.** Composition médicinale, où la composition médicale comprend
un liposome fait de phosphatidylcholine hydrogénée et de cholestérol et dont la surface a été modifiée avec un dérivé du polyéthylèneglycol, où le dérivé du polyéthylèneglycol est la polyéthylèneglycol-phosphatidyléthanolamine, et a une masse moléculaire du polyéthylèneglycol du dérivé du polyéthylèneglycol de 5000 daltons, et
un polyéthylèneglycol, où la masse moléculaire du polyéthylèneglycol est dans la plage de 400 à 4000 daltons.

**2.** Composition médicinale selon la revendication 1, où la composition médicale comprend
un liposome fait de phosphatidylcholine de soja hydrogénée et de cholestérol, et dont la surface a été modifiée avec la polyéthylèneglycol-phosphatidyléthanolamine, où la masse moléculaire du polyéthylèneglycol de la polyéthylèneglycol-phosphatidyléthanolamine est 5000 daltons, et
un polyéthylèneglycol ayant une masse moléculaire de 400 daltons.

**3.** Composition médicinale selon la revendication 1 ou 2 destinée à être utilisée dans l'inhibition de réactions anaphylactoïdes/anaphylactiques.

**4.** Composition médicinale selon l'une quelconque des revendications 1 à 3, où ledit liposome dont la surface a été modifiée avec ledit dérivé du polyéthylèneglycol et en outre ledit polyéthylèneglycol sont dispersés dans un diluant.

**5.** Composition médicinale selon l'une quelconque des revendications 1 à 4, où ledit liposome est un vecteur de médicament supportant un médicament dans un vecteur dont la surface a été modifiée avec ledit dérivé du polyéthylèneglycol.

**6.** Composition médicinale selon l'une quelconque des revendications 1 à 5, où ledit liposome et ledit polyéthylèneglycol sont dispersés dans l'eau ou le sérum physiologique, respectivement.

**7.** Préparation destinée à être utilisée dans l'inhibition de réactions anaphylactoïdes/anaphylactiques induites par le liposome tel que défini dans la revendication 1 ou 2, où la préparation comprend du polyéthylèneglycol, où la masse moléculaire du polyéthylèneglycol est dans la plage de 400 à 4000 daltons.

**8.** Préparation selon la revendication 7, où ledit polyéthylèneglycol est dispersé dans l'eau ou le sérum physiologique.

# F I G . 1

# F I G . 2

# FIG.3

# FIG.4

# F I G . 5

Legend:
- ●(UNIT MOLAR RATIO) =0
- ■(UNIT MOLAR RATIO) =0.1
- ▲(UNIT MOLAR RATIO) =1
- ✕(UNIT MOLAR RATIO) =10

Y-axis: RELEASE RATE (%)
X-axis: ACCELERATED PERIOD AT 40 °C (W)

# F I G . 6

Legend:
- ●(UNIT MOLAR RATIO) =0
- ■(UNIT MOLAR RATIO) =0.1
- ▲(UNIT MOLAR RATIO) =1
- ✕(UNIT MOLAR RATIO) =10

Y-axis: PARTICLE SIZE (nm)
X-axis: ACCELERATED PERIOD AT 40 °C (W)

# FIG.7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI5505173 B
- JP HEI720857 B
- JP 2667051 B
- WO 2004078121 A2
- EP 1174126 A1
- US 5213804 A
- JP SHO61161246 B
- JP HEI5508626 B
- JP HEI2292246 B
- JP HEI4108391 B
- WO 9742166 A

**Non-patent literature cited in the description**

- **D.D. LASIC.** LIPOSOMES from Physics to Applications. Elsevier, 1993
- Long Circulating Liposomes: Old Drugs, New Therapeutics. Springer, 1997
- Medical Applications of LIPOSOMES. Elsevier, 1998
- Drug Interview Form. Chugai Pharmaceutical Co., Ltd, December 2003
- Joint WHO/IABS Symposium on the Standardization of Albumin, Plasma Substitutes and Plasmapheresis. Develop. boil. Standard. S. Karger, 1980, vol. 48, 179-189
- **TERADA ; YOSHIMURA et al.** Liposomes in Life Science. Springer/Fairlark, 1992
- Liposome Technology Liposome Preparation and Related Techniques. CRC Press, vol. I-III